# FASCICULE DE BREVET EUROPEEN

(11) **EP 1 638 991 B1**
(45) Date de publication et mention de la délivrance du brevet: **02.08.2017**
(21) Numéro de dépôt: 04742695.2
(22) Date de dépôt: 11.05.2004
(51) Int. Cl.: C07K 7/06

(54) **COMPOSITION COSMETIQUE OU DERMOPHARMACEUTIQUE POUR REDUIRE LES SIGNES DU VIEILLISSEMENT CUTANE**
KOSMETISCHE ODER DERMOPHARMAZEUTISCHE ZUSAMMENSETZUNG ZUR VERMINDERUNG DER HAUTALTERUNGSERSCHEINUNGEN
COSMETIC OR DERMOPHARMACEUTICAL COMPOSITION FOR REDUCING THE SIGNS OF CUTANEOUS AGEING

(30) Priorité: 12.05.2003 FR 0305707; 19.12.2003 US 742344
(43) Date de publication de la demande: 29.03.2006
(73) Titulaire: Sederma, 78612 Le Perray-en-Yvelines Cedex (FR)
(72) Inventeur: LINTNER, Karl, 06500 Menton (FR)
(86) Numéro de dépôt international: PCT/FR2004/001139
(87) Numéro de publication internationale: WO 2004/101609

(56) Documents cités:
- WO-A-00/15188
- WO-A-01/91700
- US-A1- 2002 197 219
- DATABASE WPI Section Ch, Week 199907 Derwent Publications Ltd., London, GB; Class B04, AN 1999-076400 XP002303165 & JP 10 316581 A (KURARAY CO LTD) 2 décembre 1998 (1998-12-02)

## Description

La présente invention concerne des dérivés peptidiques, les compositions cosmétiques ou dermopharmaceutiques à usage topique les contenant, et l'utilisation de ces compositions pour prévenir les symptômes du vieillissement cutané et stimuler la cicatrisation cutanée. Les dérivés peptidiques, fragments de l'élastine, sont employés en association avec d'autres ingrédients actifs, dans un support cosmétiquement acceptable.

Notre peau est la première image que chacun de nous offre au regard des autres. De tout temps, son aspect a été un sujet de préoccupation.

La connaissance actuelle de sa physiologie permet maintenant de proposer des solutions cosmétiques aux différents dysfonctionnements induits par les agressions extérieures ou par le vieillissement. Beaucoup de choses restent pourtant inconnues, mal comprises et mal maîtrisées.

Il en est ainsi, par exemple, du symptôme général du vieillissement cutané, qui se traduit par l'apparition des rides, de la peau sèche, rugueuse, amincie, flasque. Le traitement de ces symptômes est un sujet important pour la recherche et le marché cosmétique.

Des facteurs externes ou internes conduisent à l'apparition des symptômes de vieillissement : la synthèse de collagène ou d'autres macromolécules du tissu conjonctif est ralentie, la protéolyse, induite par le rayonnement solaire, est accélérée : la peau s'amincit, se dessèche, perd de l'élasticité.

De nombreuses compositions cosmétiques destinées à améliorer l'aspect de la peau du visage ont été proposées à ce jour. Les produits hydratants, les crèmes anti-rides, les lotions calmantes et lissantes en font partie. Souvent, néanmoins, ces produits possèdent des effets secondaires, posent des problèmes de stabilité, et ne tiennent pas leurs promesses dans le temps. C'est notamment le cas des formules contenant des vitamines et des extraits de plantes.

La présente invention vise à résoudre le problème esthétique posé par ces symptômes et ces problèmes techniques.

Quelques peptides et dérivés peptidiques ont été déjà décrits dans le contexte d'utilisation cosmétique, comme dans K. Lintner et O. Peschard : « Biologically active peptides », lnt. J. Cosm. Sci. 22, 207-218, 2000.

Les compositions cosmétiques contenant des dérivés peptidiques correspondant à la structure générale des oligopeptides de la formule **I** :
R¹-(AA)ₙ-Val-Gly-Val-Ala-Pro-Gly-R (SEQ ID NO : 1, SEQ ID NO : 2, SEQ ID NO : 3, SEQ ID NO : 4) où (AA)ₙ est une chaîne peptidique avec (AA) un acide aminé ou un dérivé d'acide aminé,
   où "*n*" est compris entre 0 et 3 et
   où R¹ est H,
      - ou une chaîne alcoyle en C₂ à C₂₂, linéaire ou branchée, saturée ou non, hydroxylée ou non, soufrée ou non
      - ou le groupe biotinyle
      et
      R est OR² ou NR³R⁴,
      R² étant H ou une chaîne alkyle de C₁ à C₂₄, préférentiellement C₁ à C₃ ou encore C₁₄ à C₁₈,
      R³ et R⁴ étant, indépendamment l'un de l'autre, H ou une chaîne alkyle de 1 à 12 atomes de carbone
possèdent une activité dans la lutte contre les symptômes du vieillissement cutané. Toutes ces molécules, à l'exception de celles où l'on a simultanément R¹=H, R = OH et n=0, font partie de l'objet de la présente invention.

Ainsi, conformément à l'objet de la présente invention, il est maintenant proposé de nouvelles compositions cosmétiques et dermopharmaceutiques, en particulier de soins de la peau du visage, selon la revendication 1.

L'effet cosmétique recherché par l'utilisation de ces compositions cosmétiques et dermopharmaceutiques peut être renforcé par l'utilisation conjointe de tout autre ingrédient actif, synergique ou non, pour réduire les rides, augmenter l'hydratation de la peau, renforcer sa fermeté, l'épaissir et la rendre plus attractive de toute façon. Aucune composition cosmétique actuelle ne présente les avantages de la présente invention.

La présente invention a également pour objet l'utilisation de telles compositions cosmétiques ou dermopharmaceutiques destinées aux soins de la peau ou du cuir chevelu, en particulier le traitement des symptômes du vieillissement et la stimulation de la cicatrisation cutanée.

Les céramides sont des molécules lipidiques importantes de l'épiderme. Les céramides sont une classe de composés aussi connus pour être utilisés dans les produits de soin de la peau. Il a été découvert que l'utilisation conjointe des céramides et peptides selon les compositions de l'invention renforce de manière synergique l'activité anti-ride des compositions cosmétiques formulées avec le peptide seul. Ceci est un résultat particulièrement surprenant étant donné que les céramides sont habituellement utilisés pour le traitement de la peau sèche et gercée.

La présente invention décrit également un procédé de traitement cosmétique de la peau, en particulier des rides et des symptômes du vieillissement, qui consiste essentiellement à appliquer sur les zones concernées une quantité efficace d'une composition conforme à l'invention. D'autres caractéristiques, aspects et avantages de la présente invention apparaîtront à la lecture de la description détaillée qui va suivre.

### Description détaillée de l'invention :

Le peptide Val-Gly-Val-Ala-Pro-Gly est connu en tant que fragment de la protéine élastine. Il représente la séquence répétitive la plus fréquente au sein de cette protéine ; son activité chimiotactique (la propriété d'attirer les fibroblastes à un site d'inflammation ou de cicatrisation) a été décrite (cf. Senior et al. J. Cell Biol. 99, 870-874, 1984). La dérivatisation de ce peptide pour aboutir à des structures plus lipophiles permet d'augmenter considérablement le pouvoir de pénétration cutané de ces dérivés peptidiques ainsi obtenus et donc de potentialiser, voire d'initier l'activité cosmétique qui nécessite l'acheminement du dérivé peptidique vers les tissus vivants de la peau. Au cours des recherches de la présente invention, il a été montré que ce peptide, en particulier sa forme dérivée Palmitoyl-Val-Gly-Val-Ala-Pro-Gly-OH (SEQ ID NO : 6), (encore notée Pal- Val-Gly-Val-Ala-Pro-Gly-OH, Pal- Val-Gly-Val-Ala-Pro-Gly, Pal-VGVAPG), possède des activités cosmétiques insoupçonnées, à savoir un effet raffermissant et restructurant de la peau du cou et du visage. Par son effet restructurant, il contribue également à une meilleure hydratation du tégument.

Ces propriétés sont améliorées par la combinaison avec les céramides selon l'invention. Les céramides sont une classe de lipides complexes, découverts dans les couches supérieures de l'épiderme (cf. par exemple Wertz et al., J. Invest. Dermatol. 84, 410-412, 1985). Des céramides décrits dans Wertz sont les céramides 1, 3 et 4-7. Les céramides ont la Formule générale A suivante : 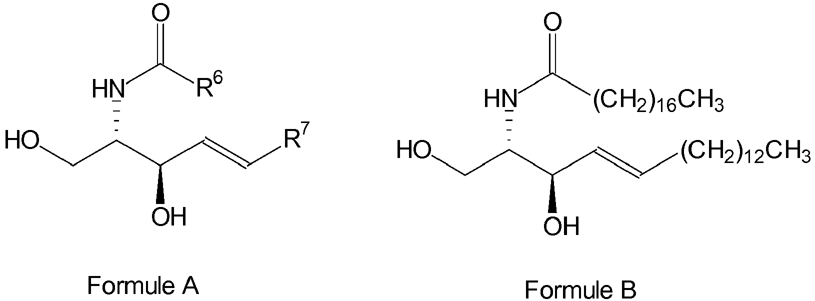 quoiqu'il en soit, cette structure basique peut être modifiée et dérivée, comme par exemple dans la Formule B. Le céramide N-Acyl-D-*erythro*-sphingosine est un composant structurel des glycolipides des mammifères, et les phospholipides, la sphingomyeline. D'autres céramides incluent le trihydroxypalmitamidohydroxypropylmyristyl éther, la n-stéaroyl-dihydrosphingosine et le palmitamido myristyl sérinate. D'autres céramides utiles selon l'invention incluent les céramides de la structure ci-dessus (Formule A) où le groupe acyle R⁶ (representé dans la Formule B comme ayant un groupe -(CH₂)₁₆CH₃) est une chaîne d'acides gras de C₁₄-C₂₂. R⁷ dans la Formule A peut être identique ou différent et est une chaîne d'acide gras de C₁₄-C₂₂. La chaîne grasse peut être saturée ou insaturée, substituée ou non substituée, linéaire ou branchée. Les céramides où R⁶ a 10 atomes de carbone ou moins ne sont pas préférés.

Beaucoup de travaux ont été consacrés à leur obtention (extraction, synthèse) et leur utilisation cosmétique. Les céramides renforcent la barrière cutanée et régulent le flux d'eau à travers la couche cornée (par exemple cf. Lintner et al. Int. J. Cosmet. Sci 19, 15-25, 1997). Il a été maintenant découvert que l'utilisation conjointe des peptides et des céramides selon l'invention dans des compositions cosmétiques ou dermopharmaceutiques augmente l'effet anti-âge des peptides objets de la présente invention considérablement, et réduit considérablement les signes du vieillissement. En particulier ces compositions cosmétiques peuvent être utilisées pour traiter et prévenir les rides. Cela signifie que le peptide et/ou le dérivé peptidique a une augmentation objectivement mesurable de son efficacité contre certains aspects du vieillissement lorsqu'il est utilisé avec un céramide. Il peut s'agir, par exemple, d'une meilleure réduction des rides, une puissance augmentée, la possibilité de stimuler ou d'inhiber au moins un procédé biochimique améliorant l'état de la peau. Le céramide est présent en quantité suffisante pour apporter une amélioration dans l'activité anti-âge du peptide et/ou du dérivé peptidique.

Pour réaliser l'invention, il suffit d'incorporer les composés actifs à des concentrations suffisantes et efficaces dans des compositions cosmétiques ou dermopharmaceutiques acceptables et d'en appliquer une quantité suffisante et efficace sur les parties du visage concernées, pendant une période allant de 2 semaines à 2 mois ou plus.

Les peptides et dérivés peptidiques fragments de l'élastine peuvent être obtenus soit par synthèse chimique classique (en phase hétérogène ou en phase homogène), soit par synthèse enzymatique (Kullman et al., J. Biol. Chem. 255, 8234, 1980) à partir des acides aminés constitutifs ou de leurs dérivés.

Pour améliorer la bio-disponibilité et le passage cutané de ces peptides on peut augmenter leur lipophilie soit par l'estérification du groupe carboxyle avec un alcool, linéaire ou branché, saturé ou non, hydroxylé ou non, conduisant aux composés H-X-Y-OR², avec R²=une chaîne alkyle de C₁ à C₂₄, préférentiellement soit C₁ à C₃, soit C₁₄ à C₁₈, soit par amidation avec une amine conduisant aux composés H-X-Y-NR³R⁴, et avec R³ et R⁴ étant indépendamment l'un de l'autre H ou une chaîne alkyle de 1 à 12 atomes de carbone, préférentiellement de 1 à 3 atomes de carbone.

Entre ainsi dans l'objet de la présente invention tout composé de formule **I** :
R¹-(AA)ₙ-Val-Gly-Val-Ala-Pro-Gly-R (SEQ ID NO : 1, SEQ ID NO : 2, SEQ ID NO : 3, SEQ ID NO : 4) où (AA)ₙ est une chaîne peptidique avec (AA) un acide aminé ou un dérivé d'acide aminé,
   où "n" est compris entre 0 et 3 et
   où R¹ est H
      - ou une chaîne alcoyle en C₂ à C₂₂, linéaire ou branchée, saturée ou non, hydroxylée ou non, soufrée ou non
      - ou le groupe biotinyle
      et
      R est OR² ou NR³R⁴,
      R² étant H ou une chaîne alkyle de C₁ à C₂₄, préférentiellement C₁ à C₃ ou encore C₁₄ à C₁₈,
      R³ et R⁴ étant, indépendamment l'un de l'autre, H ou une chaîne alkyle de 1 à 12 atomes de carbone,
      à l'exception de celui où l'on a simultanément R¹ = H, R = OH et n = 0.

Dans un mode de réalisation préféré de l'invention, R¹ est une chaîne lauroyle (C₁₂) (SEQ ID NO : 7, SEQ ID NO : 8), ou myristoyle (C₁₄) (SEQ ID NO : 9, SEQ ID NO : 10) ou stéaroyle (C₁₈) (SEQ ID NO : 11, SEQ ID NO : 12), ou oléoyle (C_{18:1}) (SEQ ID NO : 13, SEQ ID NO : 14), ou arachidique (C₂₀) (SEQ ID NO : 15, SEQ ID NO : 16), ou linoléoyle (C_{18:2}) (SEQ ID NO : 17, SEQ ID NO : 18), avec n=0 ou 1 et R² est H ou méthyle, ou éthyle, ou R est NR³R⁴ avec R³ = R⁴ = H ou méthyle.

Les dérivés peptidiques selon la présente invention comprennent, *inter alia,* les dérivés acyles, par exemple les groupes qui sont dérivés de l'acide acétique, l'acide caprique, l'acide laurique, l'acide myristique, l'acide octanoique, l'acide palmitique, l'acide stéarique, l'acide béhénique, l'acide linoléique, l'acide linolénique, l'acide oléique, l'acide isostéarique, l'acide élaidoique, l'acide 2-éthylhexaneique, l'acide gras d'huile de coco, l'aide gras d'huile de palme, l'acide gras de lanoline. Des exemples préférés de groupes acyles incluent le groupe acétyle, le groupe palmitoyle, le groupe élaidoyle, le groupe myristyle, le groupe biotinyle et le groupe octanoyle.

Les peptides et dérivés peptidiques peuvent donc être obtenus par synthèse chimique, mais également par fermentation d'une souche de bactéries, modifiées ou non par génie génétique, pour produire les séquences recherchées ou leurs différents fragments.

Enfin, les peptides peuvent être obtenus par extraction de protéines d'origine animale ou végétale, susceptibles de contenir ces séquences au sein de leur structure, suivie d'une hydrolyse contrôlée, enzymatique ou non, qui libère le fragment peptidique de séquence (AA)ₙ-Val-Gly-Val-Ala-Pro-Gly où n = 0 à 3 (SEQ ID NO : 1, SEQ ID NO : 2, SEQ ID NO : 3, SEQ ID NO : 4).

Pour réaliser l'invention, il est possible, mais non nécessaire, d'extraire soit les protéines concernées d'abord et de les hydrolyser ensuite, soit d'effectuer l'hydrolyse d'abord sur un extrait brut et de purifier les fragments peptidiques ensuite. On peut également utiliser l'hydrolysat sans en extraire les fragments peptidiques en question, en s'assurant toutefois d'avoir arrêté la réaction enzymatique d'hydrolyse à temps et de doser la présence des peptides en question par des moyens analytiques appropriés (traçage par radioactivité, immunofluorescence ou immunoprécipitation avec des anticorps spécifiques, etc.).

D'autres procédés plus simples ou plus complexes, conduisant à des produits moins chers ou plus purs sont facilement envisageables par l'homme de l'art connaissant le métier d'extraction et de purification des protéines et peptides.

Les peptides suivants représentent une sélection non limitante d'analogues de peptides de 6 ou plus aminoacides Elaidoyl-Val-Gly-Val-Ala-Pro-Gly (SEQ ID NO: 19), Palmitoyl-Ala-Val-Gly-Val-Ala-Pro-Gly (SEQ ID NO: 20), Acetyl-Ile-Ala-Val-Gly-Val-Ala-Pro-Gly (SEQ ID NO: 21). Les combinaisons particulièrement préférées contiennent les peptides N-acyl-Val-Gly-Val-Ala-Pro-Gly (SEQ ID NO: 4), encore plus particulièrement Palmitoyl-Val-Gly-Val-Ala-Pro-Gly (SEQ ID NO: 6).

Les peptides fragments de l'élastine ou leurs dérivés décrits ci-dessus sont employés dans les compositions cosmétiques conformes à l'invention à des concentrations qui peuvent varier entre 0,00001% (p/p) et 10% (p/p), préférentiellement entre 0,0001% (p/p) et 1% (p/p) ("p/p" est en poids par rapport au poids total de la composition). Un autre intervalle utile est entre 0,001 et 5% (p/p). Un autre intervalle préféré est 1 ppm à 500 ppm environ.

Selon la présente invention, la composition cosmétique ou dermopharmaceutique inclut un ou plusieurs composés peptidiques ou dérivés selon la formule **I** et un céramide selon la revendication 1.

La combinaison des peptides objets de l'invention avec les céramides nécessite des concentrations en céramide variant entre 0,0001% et 10% (p/p) pour le ou les céramides, préférentiellement entre 0,001 et 1% (p/p). Dans une autre combinaison préférée, le peptide est présent dans une quantité entre 100 et 400 ppm (p/p) environ, et le céramide entre 1 et 8% environ (p/p).

Préférentiellement, R¹ est un groupe acyle comme un groupe Palmitoyle et R² est H.

L'invention concerne l'association au peptide selon l'invention d'un céramide selon l'invention qui, lorsqu'il est combiné avec un ou plusieurs peptides, peut apporter une activité additionnelle en terme d'atténuation d'un ou plusieurs des signes du vieillissement, et en particulier l'amélioration de l'activité anti-rides est attendue. En particulier, une quantité efficace de céramides selon l'invention, notamment la N-acyl-sphingosine et la N-acyl-Dihydrosphingosine (aussi appelée N-acyl-sphinganine), leurs analogues et dérivés. Plus particulièrement, la N-stearoyl-sphinganine est préférée.

Selon un mode de réalisation particulier de l'invention, les compositions cosmétiques contiennent le peptide Palmitoyl-Val-Gly-Val-Ala-Pro-GlyOH (SEQ ID NO: 6) à la concentration variant entre 0,0001% (p/p) et 0,01% (p/p) et le céramide sous forme de N-stéaroyl-dihydrosphingosine entre 0,001% et 10,0% (p/p). Un autre intervalle utile est entre 0,001 et 5% (p/p)p our le peptide, et entre 0,01 et 1,0% pour le céramide (p/p). Dans une réalisation particulière de l'invention, la quantité d'hexapeptide est 0,002%, et la quantité de céramide 2 est 4% (p/p). Une formulation préférée est réalisée sur base huileuse.

Les compositions préférées commercialement disponibles contenant un dérivé hexapeptide sont BIOBUSTYL et BIOPEPTIDE EL, disponibles chez SEDERMA, France, qui contiennent entre 10 et 500 ppm de palmitoyl-Val-Gly-Val-Ala-Pro-Gly (SEQ ID NO: 6) et d'autres ingrédients, comme un excipient. DERMAXYL® (marque déposée), un autre produit disponible avant la publication, peut aussi être utilisé et il contient environ 200 ppm (p/p) (0,002% p/p) Pal-Val-Gly-Val-Ala-Pro-Gly (SEQ ID NO: 6) et environ 4% (p/p) du ceramide 2 dans une base huileuse. Le céramide 2 est disponible commercialement dans un produit appelé CERAMIDE 2, qui est 100% N-stearoylsphinganine, aussi connu comme N-stearoyl-dihydrosphingosine. Ces compositions peuvent être utilisées pour fabriquer les compositions selon la présente invention.

Spécifiquement, la combinaison des peptides objets de la présente invention avec d'autres ingrédients actifs cosmétiques (*vide infra*) est une réalisation avantageuse de l'invention.

L'utilisation des peptides selon l'invention en combinaison avec un ou plusieurs céramides selon l'invention sont particulièrement avantageuses pour les produits de soin de la peau destinés à réduire les signes visibles des rides, que ce soit d'une façon transitoire ou prolongée. Aussi, les compositions préférées sont des produits anti-rides pour une application topique sur la peau, et plus particulièrement le visage et les mains. Quoiqu'il en soit, tout peptide selon l'invention en combinaison avec un céramide selon l'invention, peut être ustilisé dans des produits comme des shampooings, conditionneurs ou nettoyants pour plusieurs raisons. Ils peuvent être utilisés dans ces produits pour supplémenter le traitement anti-rides obtenu par l'utilisation de produits anti-rides plus traditionnels. Ils peuvent aussi être la première raison d'appliquer ces agents anti-rides. Quoiqu'il en soit, parce que ces peptides et leurs mélanges avec des céramides peuvent avoir d'autres propriétés désirables, ils peuvent être utilisés dans des champooings, conditionneurs, produits UV-protecteurs, gels coiffants et les autres types de produits décrits ci-dessous pour des raisons complètement non associées avec leurs propriétés anti-rides.

Les Matrikines sont des fragments de peptides, dont la séquence est généralement inférieure ou égale à 20 acides aminés, issus de la protéolyse matricielle lors du nettoyage de la plaie cutanée avant cicatrisation.

L'hexapeptide VGVAPG, issu de l'hydrolyse de l'élastine par l'élastase, est une MATRIKINE, capable de rétro-agir sur les processus de renouvellement du tissu connectif et sur la prolifération cellulaire, actions particulièrement intéressantes pour la cicatrisation cutanée.

Le processus de cicatrisation fait appel à un grand nombre de cellules différentes : granulocytes, mastocytes, cellules endothéliales, plaquettes, kératinocytes, fibroblastes et monocytes. Comme toutes ces cellules ne sont pas présentes au même endroit endommagé, leur arrivée au site de la lésion doit être coordonnée dans le temps. Un des mécanismes de la signalisation aux cellules s'appelle chimiotaxie : un signal chimique attire les cellules cibles vers le site où leur présence est requise. Les cellules reconnaissent la molécule chimiotactique et migrent physiquement dans la direction du gradient de concentration. Ces molécules chimiotactiques peuvent être diverses (cytokines, peptides de courte séquence, TNF-α).

Le Palmitoyl-VGVAPG (SEQ ID NO: 6) présente les propriétés attendues d'une Matrikine : chimiotactisme, prolifération cellulaire et mobilisation calcique, remaniements matriciels via la régulation des Métalloprotéinases. Ces propriétés sont exploitées dans une application cosmétique de traitement anti-âge, pour la reconstruction d'un derme plus dense et plus élastique.

Il a ainsi été découvert de façon surprenante que certains composés de formule **I** ont des propriétés chimiotactiques. La présente invention concerne donc également l'utilisation des compositions cosmétiques ou dermopharmaceutiques selon l'invention comprenant un ou plusieurs des composés de formule **I** en vue de stimuler la cicatrisation cutanée, la restructuration et la reconstruction du derme. En particulier, l'invention a pour objet l'utilisation des compositions cosmétiques ou dermopharmaceutiques selon l'invention comprenant au moins un composé de formule **I** ayant des propriétés chimiotactiques. Encore plus particulièrement, le composé utilisé en vue de stimuler la cicatrisation cutanée peut être de formule **I** avec R¹ étant un radical palmitoyl.

Par exemple, une combinaison selon l'invention permet de potentialiser l'efficacité du dérivé peptidique Pal-VGVAPG (Matrikine de l'élastine) (SEQ ID NO: 6) par l'adjonction d'un céramide naturel et majoritaire : le céramide 2 impliqué dans le maintien hydrique ainsi que dans la cohésion et le renouvellement de l'épiderme, tout en ayant une fonction annexe protectrice des kératinocytes. Cette association permet de compléter l'effet épidermique par un effet restructurant dermique, via les propriétés chimiotactiques, prolifératrices de fibroblaste et stimulantes pour la réorganisation du tissu matriciel.

Les compositions selon l'invention sont par exemple des lotions, des laits ou des crèmes émollientes, des laits ou des crèmes pour les soins de la peau ou des cheveux, des crèmes, des lotions ou des laits démaquillants, des bases de fond de teint, des lotions, des laits ou des crèmes anti-solaires, des lotions, des laits ou des crèmes de bronzage artificiel, des crèmes ou des mousses de rasage, des lotions après-rasage, des shampooings, des rouges à lèvres, des mascaras ou des vernis à ongles.

Ces compositions peuvent également se présenter sous la forme de bâtons pour les lèvres destinés soit à les colorer, soit à éviter les gerçures, ou, en particulier, sous forme de produits de maquillage pour les yeux ou des fards et fonds de teint pour le visage.

Lorsque les compositions selon l'invention se présentent sous la forme d'émulsions de type eau-dans-l'huile ou huile-dans-l'eau, la phase grasse est essentiellement constituée d'un mélange de corps gras d'extraction ou de synthèse, avec au moins une huile, et éventuellement un autre corps gras. La phase grasse des émulsions peut constituer de 5 à 60% du poids total de l'émulsion.

La phase aqueuse desdites émulsions constitue de préférence de 30 à 85% du poids total de l'émulsion. La proportion de l'agent émulsionnant peut être comprise entre 1 et 20%, et de préférence entre 2 et 12% du poids total de l'émulsion. Lorsque les compositions selon l'invention se présentent sous forme de lotions huileuses, oléoalcooliques ou hydroalcooliques, elles peuvent constituer, par exemple, des lotions antisolaires contenant un filtre absorbant les rayons UV, des lotions adoucissantes pour la peau ; les lotions huileuses peuvent en outre constituer des huiles moussantes contenant un tensioactif oléosoluble, des huiles pour le bain, etc.

Parmi les principaux adjuvants pouvant être présents dans les compositions selon l'invention, on peut citer les solvants organiques ou hydroglycoliques, y compris le MP-diol et les polyglycérines, les corps gras d'extraction ou de synthèse, les épaississants ioniques ou non ioniques, les adoucissants, opacifiants, stabilisants, émollients, les silicones, α- ou ß-hydroxyacides, les agents anti-mousse, les agents hydratants, les vitamines, parfums, conservateurs, séquestrants, colorants, les polymères gélifiants et viscosants, les tensioactifs et émulsifiants, les autres principes actifs hydro- ou liposolubles, les extraits végétaux, extraits tissulaires, extraits marins, filtres solaires, les antioxydants.

Les mono-ou poly-alcools plus particulièrement préférés sont choisis parmi l'éthanol, l'isopropanol, le propylène-glycol, le glycérol et le sorbitol.

A titre de corps gras, parmi les huiles minérales, on peut citer l'huile de vaseline ; parmi les huiles animales, les huiles de baleine, de requin, de phoque, de menhaden, de foie de flétan, de morue, de thon, de tortue, de pied de boeuf, de pied de cheval, de pied de mouton, de vison, de loutre, de marmotte, etc. ; parmi les huiles végétales, les huiles d'amande, de germes de blé, de jojoba, de sésame, de tournesol, de palme, de noix, de karité, de shoréa, de macadamia, de pépins de cassis et similaires.

Parmi les esters d'acides gras, on peut utiliser des esters d'acide en C₁₂ à C₂₂ saturés ou insaturés et d'alcools inférieurs comme l'isopropanol ou le glycérol ou d'alcools gras en C₈ à C₂₂, linéaires ou ramifiés, saturés ou insaturés ou encore d'alcanediols-1,2 en C₁₀-C₂₂.

On peut également citer comme corps gras, la vaseline, la paraffine, la lanoline, la lanoline hydrogénée, le suif, la lanoline acetylée, les huiles de silicone.

Parmi les cires, on peut citer la cire de Sipol, la cire de lanoline, la cire d'abeille, la cire de Candelila, la cire monocristalline, la cire de Carnauba, le spermaceti, le beurre de cacao, le beurre de karité, les cires de silicone, les huiles hydrogénées concrètes à 25°C, les sucroglycérides, les oléates, myristates, linoléates et stéarates de calcium, magnésium et aluminium.

Parmi les alcools gras, on peut citer les alcools laurique, cétylique, myristique, stéarique, palmitique, oléique, et les alcools de GUERBET comme le 2-décyltétradécanol ou le 2-hexyldécanol. A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de glycérol, les alcools en C₁₂-C₁₈ comportant de 2 à 10 moles de glycérol. Il peut être aussi utile d'utiliser des épaississants tels que les dérivés de cellulose, les dérivés de d'acide polyacrylique, les gommes de guar ou de caroube ou la gomme de xanthane.

La composition selon l'invention peut également contenir des adjuvants habituellement utilisés en cosmétique ou en dermatologie et notamment des produits hydratants, des adoucissants, des produits pour le traitement d'affections cutanées, des filtres solaires, des germicides, des colorants, des conservateurs, des parfums et des propulseurs.

Lorsque les compositions selon l'invention sont des dispersions, il peut s'agir de dispersions de lécithine dans l'eau en présence de tensioactif ou encore de dispersions aqueuses de sphérules lipidiques, constituées de couches moléculaires organisées enfermant une phase aqueuse encapsulée. On peut citer, à cet effet, comme composés lipidiques, les alcools et diols à longue chaîne, les stérols tels que le cholestérol, les phospholipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras tels que le dicétylphosphate acide ou son sel de sodium, les alkylsulfates tels que le cétylsulfate de sodium, les acides gras sous forme de sels ou encore les lipides du type de ceux décrits dans les brevets français n° 2 315 991, 1 477 048 et 2 091 516 ou dans les demandes de brevet internationales WO 83/01 571 et WO 92/08685.

On peut par exemple utiliser comme autres lipides, des lipides comportant une chaîne lipophile longue contenant 12 à 30 atomes de carbone, saturée ou insaturée, ramifiée ou linéaire, par exemple une chaîne oléique, lanolique, tétradécylique, hexadécylique, isostéarylique, laurique ou alcoylphénylique. Le groupement hydrophile de ces lipides peut être un groupement ionique ou non-ionique. A titre de groupements non-ioniques, on peut citer des groupements dérivés de polyéthylèneglycol. On peut aussi utiliser avantageusement comme lipides formant la phase lamellaire, des éthers de polyglycérol tel que ceux décrits dans les brevets français n°1 477 048 , 2 091 516, 2 465 780 et 2 482 128.

A titre de groupement ionique, on peut avantageusement utiliser un groupement dérivé d'un composé amphotère, anionique ou cationique.

D'autres lipides décrits dans la demande de brevet internationale WO 83/01 571 comme pouvant être utilisés pour la formation de vésicules sont les glycolipides comme le lactosylcéramide, le galactocérébroside, les gangliosides et le trihexosylcéramide, ainsi que les phospholipides tel que le phosphatidylglycérol et le phosphatidylinositol.

En plus du peptide et/ou du dérivé peptidique et du céramide décrits ici, la composition selon l'invention peut contenir en outre un ou plusieurs ingrédients additionnels, qui peuvent être actifs, fonctionnels, habituellement utilisés dans les produits cosmétiques, de soin personnel, ou dans les produits pharmaceutiques d'application topique et/ou transdermique.

Les substances actives qui peuvent être combinées avec les peptides et dérivés peptidiques et les céramides, peuvent être des substances ayant un intérêt pharmaceutique, alimentaire ou ayant une activité cosmétique propre. Lorsqu'elles sont hydrosolubles, elles peuvent être dissoutes de façon homogène ou elles sont dans la phase aqueuse encapsulée à l'intérieur des vésicules. Les substances hydrosolubles ayant une activité cosmétique et/ou pharmaceutique peuvent être des produits destinés aux soins ou aux traitements de la peau et du cheveu tels que par exemple des humectants comme la glycérine, le sorbitol, le pentaérythritol, l'acide pyrrolidone et ses sels ; des agents de brunissage artificiel tels que la dihydroxyacétone, l'érythrulose, le glycéraldéhyde, les y-dialdéhydes tels que l'aldéhyde tartrique, ces composés étant éventuellement associés à des colorants ; des filtres solaires hydrosolubles ; des antiperspirants, des déodorants, des astringents, des produits rafraîchissants, toniques, cicatrisants, kératolytiques, dépilatoires, des eaux parfumées ; des extraits de tissus végétaux, tels que les polysaccharides , des colorants hydrosolubles ; des agents antipelliculaires ; des agents antiséborrhéiques, des oxydants tels que des agents de décoloration comme l'eau oxygénée ; des réducteurs tels que l'acide thioglycolique et ses sels.

On peut citer également les vitamines B₁ à B₁₂, C, D, H, K et leurs dérivés, les hormones peptidiques ou stéroïdiennes, les enzymes, telles que la superoxyde dismutase, les vaccins, les anti-inflammatoires stéroïdiens ou non, tels que l'hydrocortisone, les antibiotiques, les substances anti-microbiennes et bactéricides, les agents cytotoxiques ou anti-tumoraux.

Lorsque les substances actives sont liposolubles, elles peuvent se trouver incorporées dans la phase lipidique des émulsions ou dans les feuillets des vésicules tels que les liposomes, les micelles, les chylomicrons. Elles peuvent être choisies dans le groupe formé par les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes, les céramides et pseudo-céramides, ou tout lipide complexe de forme analogue aux céramides naturels de la peau..

Les peptides, objets de la présente invention peuvent être utilisés dans les compositions cosmétiques conformes à l'invention soit en ajout individuel, soit en tant que pré-mélange dans un excipient convenable, et utilisés sous forme de solution, de dispersion, d'émulsion, de pâte ou de poudre. Ils peuvent individuellement ou ensemble avec d'autres actifs, cités ou non, être véhiculés par des vecteurs cosmétiques tels que les macro-, micro- ou nanocapsules, des liposomes ou des chylomicrons, les macro-, micro- ou nanoparticules ou microéponges. Ils peuvent être également adsorbés sur des polymères organiques poudreux, les talcs, bentonites et autres supports minéraux.

Ils peuvent être utilisés dans une forme quelconque, ou sous une forme liée ou incorporée ou absorbée ou adsorbée à de macro-, micro- et nanoparticules, de macro-, micro- et nanocapsules, pour le traitement des textiles, fibres synthétiques ou naturelles, laines et tous matériaux susceptible d'être utilisés pour réaliser des vêtements et sous-vêtements de jour ou de nuit destinés à être en contact avec la peau tel que les collants, sous-vêtements, mouchoirs, lingettes, pour exercer leur effet cosmétique via ce contact textile/peau et permettre une délivrance topique continue.

A titre d'exemple illustrant l'invention, on cite quelques formules cosmétiques représentatives mais non limitatives de l'invention :

| **Exemple n°1** : Gel | g/100g |
|---|---|
| Carbomère | 0,3 |
| Propylène glycol | 2,0 |
| Glycérine | 1,0 |
| Vaseline blanche | 1,5 |
| Cylométh icone | 6,0 |
| Crodacol C90 | 0,5 |
| Lubrajel^{R} MS | 10 |
| Triéthanolamine | 0,3 |
| Palmitoyl-Val-Gly-Val-Ala-Pro-Gly-OH (SEQ ID NO: 6) | 0,0005 |
| Eau, conservateurs, parfum | qsp 100 g |

Ce gel peut être fabriqué en dissolvant le peptide dans l'eau à 80°C, en mélangeant les autres composants à 80°C, ajouter les phases l'une à l'autre, refroidir à cool to 30°C, ajouter le lubrajel, les conservateurs et le parfum.

Ce gel obtenu de façon extemporanée, peut être utilisé en application quotidienne sur la peau du visage, en particulier autour des yeux pour diminuer les infiltrations oedémateuses.

| **Exemple n°2** : Crème | g/100g |
|---|---|
| Volpo S2 | 2,4 |
| Volpo S20 | 2,6 |
| Prostéaryl 15 | 8,0 |
| Cire d'abeille | 0,5 |
| Stéaroxy dimethicone | 3,0 |
| Propylène glycol | 3,0 |
| Carbomère | 0,25 |
| Triéthanolamine | 0,25 |
| Céramide HO3 | 0,5 |
| Acetyl-Ser-Val-Gly-Val-Ala-Pro-Gly-OH (SEQ ID NO: 22) | 0,001 |
| Eau, conservateurs, parfums | qsp 100 g |

Cette émulsion est utilisée pour hydrater, restructurer et calmer la peau du visage, en particulier sur les zones à peau fragile et pour traiter les rides. Cette émulsion est préparée de la façon suivante : dissoudre le ceramide HO3 dans volpo S2, S20 et prostearyl 15 à 85°C, ajouter la cire d'abeille et la stearoxydimethicone ; mélanger les autres ingrédients dans la phase aqueuse à 75-80°C, mélanger alors les deux phases, refroidir, et ajouter le parfum. Le Ceramide HO3 est le Trihydroxypalmitamidohydroxypropyl Myristyl Ether disponible chez Sederma.

| **Exemple n°3** : Crème anti-rides | | g/100g |
|---|---|---|
| Eau déminéralisée | | qsp 100 |
| Carbomère | | 0,10 |
| Sorbate de Potassium | | 0,10 |
| Transcutol | | 3,00 |
| Glycérine | Croda | 8,00 |
| Volpo S2 [Steareth 2] | Croda | 0,60 |
| Crodafos CES [Cetearyl Alcohol | | |
| (and) Dicetyl Phosphate | | |
| (and) Ceteth 10 Phosphate] | Croda | 4,00 |
| DC 344 [Cyclomethicone] | DowCorning | 2,00 |
| Crodamol GTCC [Caprylic/Capric | | |
| Triglyceride] | Croda | 10,00 |
| Crill 3 [Sorbitan Stearate] | Croda | 1,60 |
| Parabens | | 0,30 |
| Soude 30% | | 0,35 |
| Eau déminéralisée | | 3,50 |
| DERMAXYL® | Sederma | 2,00 |

La formule ci-dessus est préparée de la façon suivante : mélanger les ingrédients huileux à 70-80°C, faire de même avec les ingrédients aqueux, ajouter ensuite pour former une émulsion. DERMAXYL® : C12-15 Alkyl benzoate C12-15 Alkyl benzoate - Tribehenin - Ceramide 2 -PEG-10 Rapeseed Sterol - Palmitoyl Oligopeptide (SEQ ID NO: 6), disponible chez Sederma

| **Exemple n°4** : Crème anti-âge apaisante | | g/100g |
|---|---|---|
| Eau déminéralisée | | qsp 100 |
| Ultrez 10 [Carbomer] | Noveon | 0,20 |
| Sorbate de Potassium | | 0,10 |
| Butylène Glycol | | 2,00 |
| Phenova [Phenoxyethanol | | |
| (and) Mixed Parabens] | Crodarom | 0,80 |
| Crill 3 [Sorbitan Stearate] | Croda | 1,00 |
| Crillet 3 [Polysorbate 60] | Croda | 2,50 |
| DC 200 (Dimethicone) | | 2,50 |
| Crodamol TN (Isotridetyl | | |
| Isononanoate) | Croda | 5,00 |
| Crodamol GTCC [Caprylic/Capric | | |
| Triglyceride] | Croda | 5,00 |
| Crodamol SS [Cetyl Esters] | Croda | 1,00 |
| Super Hartolan [Lanolin Alcohol] | Croda | 0,50 |
| Super Sterol Ester [C10-30 Cholesterol/ | | |
| Lanosterol esters] | | 0,30 |
| Crodacol CS90 [Cetearyl Alcohol] | Croda | 3,00 |
| Eau déminéralisée | | 2,50 |
| Sodium Hydroxide 38% | | 0,25 |
| CALMOSENSINE [Butylene Glycol | | |
| (and) water (and) Laureth-3 | | |
| (and) Hydroxyethylcellulose (and) | | |
| Acetyl-Dipeptide-1-cetylester] | Sederma | 4,00 |
| Palmitoyl-Val-Gly-Val-Ala-Pro-Gly (SEQ ID NO: 6) | Sederma | 0,001 |
| Ceramide 2 | | |
| (N-stearoyldihydrosphingosine) | Sederma | 0,05 |

Cette émulsion peut être préparée de la même façon que pour l'exemple précédent (mélanger la phase huileuse chauffée pré-mélangée à la phase aqueuse chauffée et prémélangée, emulsionner et refroidir).

| **Exemple n°5** : Gel anti-vergetures | | g/100g |
|---|---|---|
| Phase A | | |
| Eau déminéralisée | | qsp 100 |
| Phase B | | |
| Butylène Glycol | | 5,00 |
| Phenova [Phenoxyethanol (and) | | |
| Mixed Parabens] | Crodarom | 0,80 |
| Phase C | | |
| Crill 3 [Sorbitan Stearate] | Croda | 1,20 |
| Crillet 3 [Polysorbate 60] | Croda | 3,00 |
| DC 200 [Dimethicone] | | 2,00 |
| Crodamol IPM [Isopropyl | | |
| Myristate] | Croda | 5,00 |
| Crodamol W [Stearyl | | |
| Heptanoate] | Croda | 0,30 |
| Crodamol GTCC [Caprylic/Capric | | |
| Triglyceride] | Croda | 5,00 |
| Crodacol CS90 [Cetearyl Alcohol] | Croda | 2,00 |
| Ceramide 2 (N-stearoylsphinganine) | Sederma | 0,10 |
| Phase D | | |
| Carbopol 980 à 2% [Carbomer] | | 10,00 |
| Phase E | | |
| Sorbate de Potassium | | 0,10 |
| Phase F | | |
| Eau déminéralisée | | 2,00 |
| Sodium Hydroxide | | 0,20 |
| Phase G | | |
| Eau déminéralisée | | 10,0 |
| Pal-Val-Gly-Val-Ala-Pro-Gly (SEQ ID NO: 6) | Sederma | 0,00015 |
| ESCULOSIDE | Sederma | 0,5% |

Ce gel peut être préparé de la façon suivante : Homogénéiser la Phase B et l'ajouter à la Phase A. Chauffer la Phase (A+B) à 75°C. Chauffer les Phases C et D à 75°C. Ajouter la Phase C à la Phase (A+B), bien mélanger ; puis ajouter ma Phase D dans la phase (A+B+C). Ajouter les Phases F et E. Ajouter la Phase G à 35°C environ.

Les activités décrites au début de cette demande sont illustrées par les exemples suivants.

### Exemple n° 6 : Etude de la fermeté de la peau

Dix sujets de sexe féminin, âgés de 32 à 56 ans participent au test. La formule employée est la suivante :

| Composant | % (p/p) |
|---|---|
| Acide stéarique | 3,50 |
| Cire d'abeilles | 0,50 |
| Alcool cétylique | 1,50 |
| Octyl-palmitate | 1,50 |
| Neopentanoate d' Isostéaryl | 2,50 |
| PPG-15 stéaryl ether | 3,00 |
| Cyclométhicone | 4,50 |
| Stearoxydiméthicone | 2,00 |
| Cétéaryloctanoate | 3,00 |
| Eau déminéralisée | qsp 100 |
| Triéthanolamine 99% | 1,00 |
| Propylène glycol | 3,00 |
| Palmitoyl-Val-Gly-Val-Ala-Pro-Gly-OH (SEQ ID NO: 6) | 0,0004 |

Principe de l'étude : La sonde du Fermomètre (Courage & Khazaka) permet d'aspirer une petite zone de la peau par application répétée d'une dépression d'environ 500mbars. La déformation du tégument est suivie par la déviation d'un rayon lumineux et enregistrée sous forme d'une courbe, à partir de laquelle on peut extraire les paramètres fermeté, élasticité, tonicité cutanées.

La crème contenant le dérivé peptidique objet de la présente invention est appliquée 2 fois par jour pendant un mois sur l'avant bras. Sur le bras contralatéral, la crème « placebo », sans le peptide, est appliquée de la même façon.

On observe après un mois d'utilisation de ces crèmes que l'extensibilité de la peau diminue, que la tonicité augmente et donc que la fermeté de la peau augmente, significativement, alors que le côté placebo ne montre pas de variation significative.

### Exemple n° 7 : Etude in vitro sur les propriétés du Palmitoyl-VGVAPG (SEQ ID NO: 6)

Nous avons utilisé une approche moléculaire par analyse des gènes activés ou inhibés lorsque un épiderme est incubé en présence de pal-VGVAPG (SEQ ID NO: 6). A tout moment, les niveaux d'expression des gènes cellulaires permettent de donner une image ponctuelle de l'activité cellulaire et de mettre en évidence par comparaison à une cellule contrôle en repos, l'activation ou la répression différentielles en réponse aux conditions extérieures. La technologie des puces à ADN a permis d'étudier 400 sondes ADN codant pour 400 protéines qui couvrent les grandes fonctions des cellules de la peau. Les gènes ayant eu leur taux de production d'ARNm augmenté après l'introduction du peptide dans le milieu de culture ont été détectés par PCR (Polymerase Chain Reaction).

Les épidermes Skin-Ethic ont été pré-cultivés 24 heures en milieu approprié puis traités ou non (témoin) par le Pal-VGVAPG (SEQ ID NO: 6) pendant 4 heures. Les résultats sont exprimés en pourcentage de variation du gène considéré dans l'échantillon traité, par rapport à l'échantillon témoin. Cinq gènes sont activés positivement avec les amplitudes suivantes :

| Variation de l'expression des gènes en présence de Pal-VGVAPG (SEQ ID NO: 6) | % / témoin |
|---|---|
| Granulocyte chemotactic protein GCP2 | 227% |
| Gène récepteur à l'ephrin | 179% |
| Protéine inhibitrice du plasminogen | 166% |
| l'EGF response factor (ou ERF1) | 154% |
| Gène de la calmodulin | 150% |

Le gène le plus fortement stimulé est le gène GCP2 gouvernant la synthèse d'une protéine chimiotactique qui est impliquée dans l'attraction des cellules de nettoyage après blessures : monocytes, neutrophiles. Il est par ailleurs intéressant de constater la spécificité de ce peptide : cinq gènes spécifiques réagissent.

### Exemple n° 8 : Fond de teint hydratant et anti-rides

| Composé | % (p/p) |
|---|---|
| Eau déminéralisée | 53,36 |
| KOH 10% | 1,30 |
| Polysorbate 80 | 0,10 |
| Titanium dioxyde | 6,00 |
| Talc | 3,05 |
| Yellow Iron Oxide | 1,80 |
| Red Iron Oxide | 1,00 |
| Black Iron Oxide | 0,15 |
| Propylène glycol | 6,00 |
| Magnésium Aluminium Silicate | 1,00 |
| Sodium Carboxyméthylcellulose | 0,12 |
| DiPPG3 Myristyl Ether Adipate | 12,00 |
| Néopentanoate d' Isostearyl | 4,00 |
| Crodafos CS 20 | 4,00 |
| Stéareth-10 | 2,00 |
| Alcool Cétylique | 0,50 |
| Stéareth-2 | 0,50 |
| Ceramide 2 | 0,10 |
| Pal-Val-Gly-Val-Ala-Pro-Gly-OH (SEQ ID NO: 6) | 0,0004 |
| Conservateurs | qsp |

### Exemple n°9 : Activité anti-rides

24 personnes (âge moyen 54 ans) participent à un test d'utilisation d'une crème teintée selon l'exemple n° 8.

Les rides autour des yeux sont évaluées par auto-évaluation/questionnaire et par la technique des empreintes. Le produit est appliqué pendant 56 jours sur les zones concernées, 2 fois par jour. Les mesures sont faites à J0 et J56. En résumé, il résulte de l'étude une diminution mesurable des rides (voir tableau). Par ailleurs, cette diminution peut être observée à l'oeil nu, alors que les sites traités avec la même crème teintée sans le peptide et sans le céramide ne montrent pas d'amélioration significative des symptômes du vieillissement cutané.

| Valeurs | Moyennes | Maximales |
|---|---|---|
| Volume de la ride principale | -13,7% | -36% |
| Profondeur de la ride principale | -10,1% | -27% |
| Surface occupée par les rides profondes | -40,3% | -98% |
| Surface occupée par les rides moyennes | -24,5% | -86% |

## Revendications

1. Composition cosmétique ou dermopharmaceutique comprenant un excipient cosmétiquement acceptable et la combinaison :
- d'un ou plusieurs composés peptidiques répondant à la formule **I** : R¹-(AA)ₙ-Val-Gly-Val-Ala-Pro-Gly-R (SEQ ID NO: 1, SEQ ID NO: 2, SEQ ID NO : 3, SEQ ID NO : 4) où (AA)ₙ est une chaîne peptidique avec (AA) un acide aminé ou un dérivé d'acide aminé,
où "*n*" est compris entre 0 et 3 et
où R¹ est H,
- ou une chaîne alcoyle en C₂ à C₂₂, linéaire ou branchée, saturée ou non, hydroxylée ou non, soufrée ou non
- ou le groupe biotinyle
et
R est OR², ou NR³R⁴,
R² étant H ou une chaîne alkyle de C₁ à C₂₄, préférentiellement C₁ à C₃ ou encore C₁₄ à C₁₈,
R³ et R⁴ étant, indépendamment l'un de l'autre, H ou une chaîne alkyle de 1 à 12 atomes de carbone
à l'exception de celui où l'on a simultanément R¹ = H, R = OH et n = 0,
- et d'un ou plusieurs céramides choisis parmi la N-acyl-sphingosine, la N-acyl-Dihydrosphingosine (N-acyl-sphinganine), la N-stearoyl-dihydrosphingosine (n-Stearoylsphinganine) et le trihydroxypalmitamidohydroxypropyl Myristyl Ether.

2. Composition selon la revendication 1 où R¹ est une chaîne lauroyle (C₁₂) (SEQ ID NO : 7, SEQ ID NO : 8), ou myristoyle (C₁₄) (SEQ ID NO : 9, SEQ ID NO : 10), ou stéaroyle (C₁₈) (SEQ ID NO : 11, SEQ ID NO : 12), ou oléoyle (C_{18:1}) (SEQ ID NO: 13, SEQ ID NO: 14), ou arachidique (C₂₀) (SEQ ID NO : 15, SEQ ID NO : 16), ou linoléoyle (C_{18:2}) (SEQ ID NO : 17, SEQ ID NO : 18), avec n=0 ou 1 et R² est H ou méthyle, ou éthyle, ou R est NR³R⁴ avec R³ = R⁴ = H ou méthyle.

3. Composition cosmétique ou dermopharmaceutique selon la revendication 1 ou 2, **caractérisée en ce que** les peptides sont obtenus par synthèse chimique, par voie enzymatique, par fermentation ou par extraction de protéines d'origine végétale.

4. Composition cosmétique ou dermopharmaceutique selon l'une des revendications 1 à 3, **caractérisée en ce qu'**elle contient en outre un ou plusieurs ingrédients actifs cosmétiques choisis parmi le groupe constitué des humectants comme la glycérine, le sorbitol, le pentaérythritol, l'acide pyrrolidone et ses sels ; des agents de brunissage artificiel tels que la dihydroxyacétone, l'érythrulose, le glycéraldéhyde, les γ-dialdéhydes tels que l'aldéhyde tartrique, ces composés étant éventuellement associés à des colorants ; des filtres solaires hydrosolubles ; des antiperspirants, des déodorants, des astringents, des produits rafraîchissants, toniques, cicatrisants, kératolytiques, dépilatoires, des eaux parfumées ; des extraits de tissus végétaux, tels que les polysaccharides ; des colorants hydrosolubles ; des agents antipelliculaires ; des agents antiséborrhéiques, des oxydants tels que des agents de décoloration comme l'eau oxygénée ; des réducteurs tels que l'acide thioglycolique et ses sels, des vitamines B₁ à B₁₂, C, D, H, K et leurs dérivés, des hormones peptidiques ou stéroïdiennes, des enzymes, telles que la superoxyde dismutase, des vaccins, des anti-inflammatoires stéroïdiens ou non, tels que l'hydrocortisone, des antibiotiques, des substances anti-microbiennes et bactéricides, des agents cytotoxiques ou anti-tumoraux, des substances actives liposolubles choisies dans le groupe formé par les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes, les céramides et pseudo-céramides.

5. Composition cosmétique ou dermopharmaceutique selon l'une quelconque des revendications 1 à 4, **caractérisée en ce que** les composés peptidiques sont utilisés à des concentrations variant entre 0,00001% (p/p) et 10% (p/p).

6. Composition cosmétique ou dermopharmaceutique selon la revendication 5, **caractérisée en ce que** les concentrations en composés peptidiques varient entre 0,0001% (p/p) et 1% (p/p).

7. Composition cosmétique ou dermopharmaceutique selon l'une dermopharmaceutique selon l'une quelconque des revendications 1 à 6, **caractérisée en ce qu'**elle contient en outre un ou plusieurs céramides à une concentration variant entre 0,0001% (p/p) et 10% (p/p).

8. Composition cosmétique ou dermopharmaceutique selon la revendication 7, **caractérisée en ce que** la concentration en céramide(s) varie entre 0,001% (p/p) et 1% (p/p).

9. Composition cosmétique ou dermopharmaceutique selon l'une quelconque des revendications 1 à 8, **caractérisée en ce qu'**elle comprend un ou plusieurs composés peptidiques choisis parmi : Elaidoyl-Val-Gly-Val-Ala-Pro-Gly (SEQ ID NO: 19), Palmitoyl-Ala-Val-Gly-Val-Ala-Pro-Gly (SEQ ID NO: 20), Acetyl-Ile-Ala-Val-Gly-Val-Ala-Pro-Gly (SEQ ID NO: 21), N-acyl-Val-Gly-Val-Ala-Pro-Gly (SEQ ID NO: 4) et Palmitoyl-Val-Gly-Val-Ala-Pro-Gly (SEQ ID NO: 6).

10. Composition cosmétique ou dermopharmaceutique selon la revendication 9, **caractérisée en ce qu'**elle contient le peptide Palmitoyl-Val-Gly-Val-Ala-Pro-GlyOH (SEQ ID NO: 6) à la concentration variant entre 0,0001% (p/p) et 0,01% (p/p) et le céramide sous forme de N-stéaroyl-dihydrosphingosine entre 0,001% et 1,0%.

11. Composition cosmétique ou dermopharmaceutique selon l'une quelconque des revendications 1 à 10, **caractérisée en ce que** le(s) composé(s) est (sont) utilisé(s) sous forme de solution, de dispersion, d'émulsion, de pâte ou de poudre, individuellement ou en pré-mélange ou sont véhiculés individuellement ou en pré-mélange par des vecteurs comme les macro-, micro- ou nanocapsules, des liposomes ou des chylomicrons, des macro-, micro- ou nanoparticules, ou les microéponges, ou adsorbés sur des polymères organiques poudreux ou supports minéraux.

12. Composition cosmétique ou dermopharmaceutique selon l'une quelconque des revendications 1 à 11 dans une forme galénique choisie parmi lotions, laits ou crèmes émollients ; laits ou crèmes pour les soins de la peau ou des cheveux ; lotions ou laits démaquillants ; bases de fond de teint ; lotions, laits ou crèmes anti-solaires ; lotions, laits ou crèmes de bronzage artificiel ; crèmes ou mousses de rasage ; lotions après-rasage ; shampooings ; rouges à lèvres ; mascaras et vernis à ongles.

13. Composition cosmétique ou dermopharmaceutique selon l'une quelconque des revendications 1 à 12, **caractérisée en ce qu'**elle contient au moins un autre ingrédient habituellement utilisé en cosmétique dans un mélange cosmétiquement acceptable choisis parmi les catégories suivantes : solvants organiques ou hydroglycoliques, corps gras d'extraction ou de synthèse, épaississants ioniques ou non ioniques, adoucissants, opacifiants, stabilisants, émollients, silicones, α-hydroxyacides, agents anti-mousse, parfums, conservateurs, séquestrants, colorants, polymères gélifiants et viscosants, tensioactifs et émulsifiants.

14. Utilisation d'une composition selon l'une quelconque des revendications 1 à 13 pour un traitement topique cosmétique non-thérapeutique pour prévenir et traiter les signes du vieillissement cutané.

15. Utilisation selon la revendication 14, pour prévenir et traiter les rides.

16. Utilisation de la composition selon la revendication 10 pour un traitement topique non thérapeutique cosmétique des vergetures.

17. Composition selon l'une des revendications 1 à 13 pour un traitement pour stimuler la cicatrisation cutanée.

## Patentansprüche

1. Kosmetische oder dermopharmazeutische Zusammensetzung, umfassend einen kosmetisch akzeptablen Hilfsstoff und die Kombination:
- einer oder mehrerer Peptidverbindungen gemäß der Formel **I**:
R¹-(AA)ₙ-Val-Gly-Val-Ala-Pro-Gly-R (SEQ ID NO : 1, SEQ ID NO : 2, SEQ ID NO : 3, SEQ ID NO : 4), wobei (AA)ₙ eine Peptidkette mit (AA), eine Aminosäure oder ein Aminosäurederivat ist,
wobei "n"zwischen 0 und 3 inklusive ist und
wobei R¹ H ist,
- oder eine lineare oder verzweigte, gesättigte oder nicht, hydroxylierte oder nicht, sulfunierte oder nicht, C₂-C₂₂-Alkoylkette
- oder die Biotinylgruppe
und
R OR² oder NR³R⁴ ist,
wobei R² H oder eine C₁-C₂₄-, vorzugsweise C₁-C₃- oder auch C₁₄-C₁₈-Alkylkette ist,
R³ und R⁴ unabhängig voneinander H oder eine Alkylkette mit 1 bis 12 Kohlenstoffatomen sind, mit Ausnahme dessen, bei dem gleichzeitig R¹ = H, R = OH und n = 0 ist,
- eines oder mehrerer Ceramide, ausgewählt aus dem N-acylsphingosin, dem N-acyl-dihydrosphingosin (N-acyl-sphinganin), dem N-stearoyl-dihydrosphingosin (n-Stearoylsphinganin) und dem Trihydrxypalmitamidohydroxypropyl Myristil Ether.

2. Verbindung nach Anspruch 1, wobei R¹ eine Lauroyl- (C₁₂) (SEQ ID NO : 7, SEQ ID NO : 8) oder Myristoyl- (C₁₄) (SEQ ID NO : 9, SEQ ID NO : 10) oder Stearoyl- (C₁₈) (SEQ ID NO : 11, SEQ ID NO : 12) oder Oleoyl-(C_{18:1}) (SEQ ID NO : 13, SEQ ID NO : 14) oder Arachin- (C₂₀) (SEQ ID NO : 15, SEQ ID NO : 16) oder Linoleoylkette (C_{18 :2}) (SEQ ID NO : 17, SEQ ID NO : 18) ist mit n=0 oder 1 und R² ist H oder Methyl oder Ethyl oder R ist NR³R⁴ mit R³ = R⁴ = H oder Methyl.

3. Kosmetische oder dermopharmazeutische Zusammensetzung nach Anspruch 1 oder 2, **dadurch gekennzeichnet, dass** die Peptide durch chemische Synthese, auf enzymatischem Weg, durch Fermentierung oder durch Extraktion von Proteinen pflanzlichen Ursprungs gewonnen werden.

4. Kosmetische oder dermopharmazeutische Zusammensetzung nach einem der Ansprüche 1 bis 3, **dadurch gekennzeichnet, dass** sie ferner einen oder mehrere kosmetische Wirkstoffe enthält, die aus der Gruppe ausgewählt sind, die aus den Anfeuchtemitteln wie das Glyzerin, das Sorbitol, das Pentaerythritol, die Pyrrolidonsäure und ihre Salze; den künstlichen Bräunungsmitteln wie das Dihydroxyaceton, die Erythrulose, das Glyceraldehyd, die γ-Dialdehyde wie das Weinsteinaldehyd, wobei diese Verbindungen eventuell Farbstoffen zugeordnet sind; den wasserlöslichen Sonnenfiltern; den Antitranspiranten, den Deodorants, den Adstringenzien, den Erfrischungs-, tonisierenden, heilenden, keratolytischen, depilatorischen Produkten, den Duftwässern; den Pflanzengewebsextrakten wie die Polysaccharide; den wasserlöslichen Farbstoffen; den Anti-Schuppen-Mitteln; den antiseborrhoischen Mitteln, den Oxidantien wie die Entfärbungsmittel wie das oxygenierte Wasser; den Reduktoren wie die Thioglycolsäure und ihre Salze, den Vitaminen B₁ bis B₁₂, C, D, H, K und ihren Derivaten, den Peptid- oder Steroidhormonen, den Enzymen wie die Superoxiddismutase, den Impfstoffen, den Steroid-Entzündungshemmern oder nicht wie das Hydrocortison, den Antibiotika, den antimikrobiellen und bakteriziden Substanzen, den zytotoxischen oder Anti-Tumor-Mitteln, den fettlöslichen aktiven Substanzen, ausgewählt aus der Gruppe, die von den fettlöslichen Sonnenfiltern, den Substanzen, die zur Verbesserung des Zustands trockener oder seniler Haut bestimmt sind, den Tocopherolen, den Vitaminen E, F oder A und ihren Estern, der Retinolsäure, den Antioxidanzien, den essentiellen Fettsäuren, der Glycyrrhetinsäure, den Keratolytika und den Carotinoiden, den Ceramiden und Pseudo-Ceramiden besteht, gebildet ist.

5. Kosmetische oder dermopharmazeutische Zusammensetzung nach einem der Ansprüche 1 bis 4, **dadurch gekennzeichnet, dass** die Peptidverbindungen in Konzentrationen zwischen 0,00001 % (p/p) und 10 % (p/p) schwankend verwendet werden.

6. Kosmetische oder dermopharmazeutische Zusammensetzung nach Anspruch 5, **dadurch gekennzeichnet, dass** die Peptidverbindungskonzentrationen zwischen 0,0001 % (p/p) und 1 % (p/p) schwanken.

7. Kosmetische oder dermopharmazeutische Zusammensetzung nach einem Dermopharmazeutikum nach einem der Ansprüche 1 bis 6, **dadurch gekennzeichnet, dass** sie ferner ein oder mehrere Ceramide in einer Konzentration zwischen 0,00001 % (p/p) und 10 % (p/p) schwankend enthält.

8. Kosmetische oder dermopharmazeutische Zusammensetzung nach Anspruch 7, **dadurch gekennzeichnet, dass** die Ceramidkonzentration zwischen 0,001 % (p/p) und 1 % (p/p) schwankt.

9. Kosmetische oder dermopharmazeutische Zusammensetzung nach einem der Ansprüche 1 bis 8, **dadurch gekennzeichnet, dass** sie eine oder mehrere Peptidverbindungen umfasst, die ausgewählt sind aus Elaidoyl-Val-Gly-Val-Ala-Pro-Gly (SEQ ID NO: 19), Palmitoyl-Ala-Val-Gly-Val-Ala-Pro-Gly (SEQ ID NO: 20), Acetyl-Ile-Ala-Val-Gly-Val-Ala-Pro-Gly (SEQ ID NO: 21), N-acyl-Val-Gly-Val-Ala-Pro-Gly (SEQ ID NO: 4) und Palmitoyl-Val-Gly-Val-Ala-Pro-Gly (SEQ ID NO: 6).

10. Kosmetische oder dermopharmazeutische Zusammensetzung nach Anspruch 9, **dadurch gekennzeichnet, dass** sie das Peptid Palmitoyl-Val-Gly-Val-Ala-Pro-Gly (SEQ ID NO: 6) in der Konzentration enthält, die zwischen 0,0001 % (p/p) und 0,01 % (p/p) schwankt und das Ceramid in Form von N-stearoyl-dihydrosphingosin zwischen 0,001 % und 1,0 %.

11. Kosmetische oder dermopharmazeutische Zusammensetzung nach einem der Ansprüche 1 bis 10, **dadurch gekennzeichnet, dass** die Verbindung(en) in Form von Lösung, Dispersion, Emulsion, Paste oder Pulver, individuell oder vorgemischt verwendet wird/werden oder individuell oder vorgemischt von Vektoren wie die Makro-, Mikro- oder Nanokapseln, Liposomen oder Chylomikrone, Makro-, Mikro- oder Nanopartikel oder den Mikroschwämmen befördert wird/werden oder auf pulverförmigen organischen Polymeren oder mineralischen Trägern adsorbiert wird/werden.

12. Kosmetische oder dermopharmazeutische Zusammensetzung nach einem der Ansprüche 1 bis 11 in einer galenischen Form, die aus den Lotionen, weichmachenden Milchen oder Cremes; Milchen oder Cremes zur Pflege der Haut oder des Haars; Abschminklotionen oder -milchen; Teintfoundationgrundlagen; Sonnenschutzlotionen, -milchen oder -cremes; künstlichen Bräunungslotionen, -milchen oder -cremes; Rasiercremes oder -schäumen; Aftershave-Lotionen; Shampoons; Lippenstiften, Mascaras oder Nagellacken ausgewählt ist.

13. Kosmetische oder dermopharmazeutische Zusammensetzung nach einem der Ansprüche 1 bis 12, **dadurch gekennzeichnet, dass** sie mindestens einen anderen Inhaltsstoff enthält, der in üblicher Weise in der Kosmetik in einem kosmetisch akzeptablen Gemisch verwendet wird, ausgewählt aus den folgenden Kategorien: organische oder hydroglycolische Lösungsmittel, extrahierte oder synthetisierte Fettkörper, ionische oder nichtionische Verdickungsmittel, Weichmacher, Trübungsmittel, Stabilisatoren, Softener, Silikone, α-Hydroxysäuren, AntiSchaummittel, Duftstoffe, Konservierungsstoffe, Sequestriermittel, Farbstoffe, gelbildende und viskosierende Polymere, Tenside und Emulgatoren.

14. Verwendung einer Zusammensetzung nach einem der Ansprüche 1 bis 13 für eine nicht therapeutische kosmetische topische Behandlung zur Vorbeugung und Behandlung von Zeichen der Hautalterung.

15. Verwendung nach Anspruch 14 zur Vorbeugung und Behandlung der Falten.

16. Verwendung der Zusammensetzung nach Anspruch 10 für eine kosmetische nicht therapeutische topische Behandlung von Streifen.

17. Zusammensetzung nach einem der Ansprüche 1 bis 13 für eine Behandlung zum Stimulieren der Heilung der Haut.

## Claims

1. A cosmetic or dermopharmaceutical composition comprising a cosmetically acceptable excipient and the combination:
- Of one or more peptide compounds corresponding to formula I:
R¹-(AA)ₙ-Val-Gly-Val-Ala-Pro-Gly-R (SEQ ID NO: 1, SEQ ID NO: 2, SEQ ID NO: 3, SEQ ID NO: 4) wherein (AA)ₙ is a peptide chain with (AA) being an amino acid or an amino acid derivative,
Wherein "n" is between 0 and 3, and
Wherein R¹ is H,
- or a linear or branched, saturated or not, hydroxylated or not, sulfur-containing or not C₂ to C₂₂ alkyl chain
- or the biotinyl group
And
R is OR², or NR³R⁴,
R² being H or an alkyl chain of C₁ to C₂₄, preferably C₁ to C₃ or C₁₄ to C₁₈,
R³ and R⁴ being, independently of one another, H or an alkyl chain of 1 to 12 carbon atoms
With the exception of the peptide compound in which R¹ = H, R = OH and n = 0,
- and of one or more ceramides selected from N-acyl-sphingosine, N-acyl-dihydrosphingosine (N-acyl-sphinganine), N-stearoyl-dihydrosphingosine (n-stearoylsphinganine) and trihydroxypalmitamidohydroxypropyl myristyl Ether.

2. The composition of claim 1, wherein R¹ is a lauroyl chain (C₁₂) (SEQ ID NO: 7, SEQ ID NO: 8), or myristoyl (C₁₄) (SEQ ID NO: 9, SEQ ID NO: 10), or stearoyl (C₁₈) (SEQ ID NO: 11, SEQ ID NO: 12), or oleoyl (C_{18:1}) (SEQ ID NO: 13, SEQ ID NO: 14), or arachidic (C₂₀) (SEQ ID NO: 15, SEQ ID NO: 16), or linoleoyl (C_{18:2}) (SEQ ID NO: 17, SEQ ID NO: 18), with n = 0 or 1 and R² is H or methyl or ethyl, or R is NR³R⁴ with R³ = R⁴ = H or methyl.

3. The cosmetic or dermopharmaceutical composition according to claim 1 or 2, wherein the peptides are obtained by chemical synthesis, by enzymatic path, by fermentation or by extraction of proteins of plant origin.

4. The cosmetic or dermopharmaceutical composition according to any one of claims 1 to 3, **characterized in that** it also contains one or more cosmetic active ingredients selected from the group consisting of humectants such as glycerin, sorbitol, pentaerythritol, pyrrolidone acid and its salts; artificial tanning agents such as dihydroxyacetone, erythrulose, glyceraldehyde, γ-dialdehydes such as tartaric aldehyde, these compounds being optionally combined with dyes; water-soluble sunscreens; antiperspirants, deodorants, astringents, refreshing, tonic, healing, keratolytic, depilatory products, perfumed waters; extracts of plant tissues, such as polysaccharides; water-soluble dyes; antidandruff agents; anti-seborrhoeic agents, oxidants such as discoloration agents such as hydrogen peroxide; reducing agents such as thioglycolic acid and its salts, vitamins B1 to B12, C, D, H, K and derivatives thereof, peptide or steroid hormones, enzymes such as superoxide dismutase, vaccines, steroidal or non-steroidal antiinflammatory agents, such as hydrocortisone, antibiotics, anti-microbial and bactericidal substances, cytotoxic or anti-tumor agents, liposoluble active substances selected from the group consisting of fat-soluble sunscreens, substances aiming to improve the condition of dry or senile skins, tocopherols, vitamins E, F or A and their esters, retinoic acid, antioxidants, essential fatty acids, glycyrrhetinic acid, keratolytics and carotenoids, ceramides and pseudo-ceramides.

5. The cosmetic or dermopharmaceutical composition according to anyone of claims 1 to 4, wherein the peptide compounds are used in concentrations varying between 0.00001% (w/w) and 10% (w/w).

6. The cosmetic or dermopharmaceutical composition according to claim 5, **characterized in that** the concentrations of peptide compounds vary between 0.0001% (w/w) and 1% (w/w).

7. The cosmetic or dermopharmaceutical composition according to anyone of claims 1 to 6, **characterized in that** it further comprises one or more ceramides at a concentration ranging between 0.0001% (w/w) and 10% (w/w).

8. The cosmetic or dermopharmaceutical composition according to claim 7, **characterized in that** the ceramide concentration varies between 0.001% (w/w) and 1% (w/w).

9. The cosmetic or dermopharmaceutical composition according to any one of claims 1 to 8, **characterized in that** it comprises one or more peptide compounds selected from: Elaidoyl-Val-Gly-Val-Ala-Pro-Gly (SEQ ID NO: 19), Palmitoyl-Ala-Val-Gly-Val-Ala-Pro-Gly (SEQ ID NO: 20), Acetyl-Ile-Ala-Val-Gly-Val-Ala-Pro-Gly (SEQ ID NO: 21), N-acyl-Val-Gly-Val-Ala-Pro-Gly (SEQ ID NO: 4) and Palmitoyl-Val-Gly-Val-Ala-Pro-Gly (SEQ ID NO: 6).

10. The cosmetic or dermopharmaceutical composition according to claim 9, **characterized in that** it contains the Palmitoyl-Val-Gly-Val-Ala-Pro-Gly-OH peptide (SEQ ID NO: 6) at a concentration ranging between 0.0001% (w/w) and 0.01% (w/w) and the ceramide in the form of N-stearoyl-dihydrosphingosine between 0.001% and 1.0%.

11. Cosmetic or dermopharmaceutical composition according to any one of claims 1 to 10, **characterized in that** the compound (s) is/are used in the form of solution, dispersion, emulsion, paste or powder, individually or in premix, or are individually or pre-mixed by vectors such as macro-, micro- or nanocapsules, liposomes or chylomicrons, macro-, micro- or nanoparticles, or microponges, or adsorbed on powdery organic polymers or inorganic supports.

12. The cosmetic or dermopharmaceutical composition according to any one of claims 1 to 11 in a galenical form selected from lotions, emollient milks or creams; milks or creams for the care of the skin or hair; cleansing lotions or milk; foundation bases; sunscreen lotions, milks or creams; lotions, milks or creams for artificial tanning; shaving creams or foams; aftershave lotions; shampoos; lipsticks; mascaras and nail varnishes.

13. The cosmetic or dermopharmaceutical composition according to anyone of claims 1 to 12, **characterized in that** it contains at least one other ingredient commonly used in cosmetics in a cosmetically acceptable mixture selected from the following categories: organic or hydroglycolic solvents, fatty substances obtained by synthesis or extraction, ionic or nonionic thickeners, softeners, opacifiers, stabilizers, emollients, silicones, α-hydroxy acids, antifoam agents, perfumes, preservatives, sequestrants, dyes, gelling and viscosifying polymers, surfactants and emulsifiers.

14. Use of a composition according to any one of claims 1 to 13 for a non-therapeutic cosmetic topical treatment for preventing and treating the signs of aging of the skin.

15. Use according to claim 14 for preventing and treating wrinkles.

16. Use of the composition according to claim 10 for a topical non-therapeutic cosmetic treatment of stretch marks.

17. Composition according to one of claims 1 to 13 for a treatment to stimulate cutaneous healing.


### SEQUENCE LISTING

<110> SEDERMA
<120> Composition cosmétique ou dermopharmaceutique pour réduire les signes du vieillissement cutané
<130> VGVAPG_PCT
<140> PCT
   <141> 2004-05-10
<160> 22
<170> Patentln version 3.2
<210> 1
   <211> 7
   <212> PRT
   <213> artificial sequence
<220>
   <223> synthetic modified peptide
<220>
   <221> MISC_FEATURE
   <222> (1)..(1)
   <223> Xaa can be any natural or non-natural aminoacid
<220>
   <221> MOD_RES
   <222> (1)..(1)
   <223> AMIDATION, R1 Sequence description :R1 Xaa Val Gly Val Ala Pro Gly R with R1 = H or alkoyl chain from C2 to C22 linear or branched, saturated or not, hydroxylated or not, with sulfur or not, or biotinyl group
<220>
   <221> MISC_FEATURE
   <222> (7)..(7)
   <223> R is OR2 or NR3R4 with R2 =H or alkyl chain from C1 to C24 preferably C1 to C3 or C14 to C18 and with R3 or R4 independently = H or alkyl group from C1 to C12
<400> 1
   Xaa Val Gly Val Ala Pro Gly
   1 5
<210> 2
   <211> 8
   <212> PRT
   <213> artificial sequence
<220>
   <223> synthetic modified peptide
   <220>
   <221> MOD_RES
   <222> (1)..(1)
   <223> AMIDATION, R1 Sequence description :R1 Xaa Xaa Val Gly Val Ala Pro Gly R with R1 = H or alkoyl chain from C2 to C22 linear or branched, saturated or not, hydroxylated or not, with sulfur or not, or biotinyl group
<220>
   <221> MISC_FEATURE
   <222> (1)..(2)
   <223> Xaa can be any natural or non-natural aminoacid
<220>
   <221> MISC_FEATURE
   <222> (8)..(8)
   <223> R is OR2 or NR3R4 with R2 =H or alkyl chain from C1 to C24 preferably C1 to C3 or C14 to C18 and with R3 or R4 independently = H or alkyl group from C1 to C12
<400> 2
   Xaa Xaa Val Gly Val Ala Pro Gly
   1 5
<210> 3
   <211> 9
   <212> PRT
   <213> artificial sequence
<220>
   <223> synthetic modified peptide
<220>
   <221> MOD_RES
   <222> (1)..(1)
   <223> AMIDATION, R1 Sequence description :R1 Xaa Xaa Xaa Val Gly Val Ala Pro Gly R with R1 = H or alkoyl chain from C2 to C22 linear or branched, saturated or not, hydroxylated or not, with sulfur or not, or biotinyl group
<220>
   <221> MISC_FEATURE
   <222> (1)..(3)
   <223> Xaa can be any natural or non-natural aminoacid
<220>
   <221> MISC_FEATURE
   <222> (9)..(9)
   <223> R is OR2 or NR3R4 with R2 =H or alkyl chain from C1 to C24 preferably C1 to C3 or C14 to C18 and with R3 or R4 independently = H or alkyl group from C1 to C12
<400> 3
   Xaa Xaa Xaa Val Gly Val Ala Pro Gly
   1 5
<210> 4
   <211> 6
   <212> PRT
   <213> artificial sequence
<220>
   <223> synthetic modified peptide
<220>
   <221> MOD_RES
   <222> (1)..(1)
   <223> AMIDATION, R1 Sequence description :R1 Val Gly Val Ala Pro Gly R with R1 =H or alkoyl chain from C2 to C22 linear or branched, saturated or not, hydroxylated or not, with sulfur or not, or biotinyl group with the exception of R1=H when R=OH
<220>
   <221> MISC_FEATURE
   <222> (6)..(6)
   <223> R is OR2 or NR3R4 with R2 =H or alkyl chain from C1 to C24 preferably C1 to C3 or C14 to C18 and with R3 or R4 independently = H or alkyl group from C1 to C12
<400> 4
   Val Gly Val Ala Pro Gly
   1 5
<210> 5
   <211> 6
   <212> PRT
   <213> artificial sequence
<220>
   <223> synthetic modified peptide
<220>
   <221> MOD_RES
   <222> (1)..(1)
   <223> ACETYLATION
   sequence description : Acetyl Val Gly Val Ala Pro Gly
<400> 5
   Val Gly Val Ala Pro Gly
   1 5
<210> 6
   <211> 6
   <212> PRT
   <213> artificial sequence
   <220>
   <223> synthetic modified peptide
<220>
   <221> MOD_RES
   <222> (1)..(1)
   <223> AMIDATION, Pal (C16)
   sequence description : Palmitoyl Val Gly Val Ala Pro Gly
<400> 6
   Val Gly Val Ala Pro Gly
   1 5
<210> 7
   <211> 6
   <212> PRT
   <213> artificial sequence
<220>
   <223> synthetic modified peptide
<220>
   <221> MOD_RES
   <222> (1)..(1)
   <223> AMIDATION, Lau (C12)
   sequence description : Lauroyl Val Gly Val Ala Pro Gly R
<220>
   <221> MISC_FEATURE
   <222> (6)..(6)
   <223> R is OR2 or NR3R4 with R2 =H or methyl, ethyl and with R3 = R4 = H or methyl
<400> 7
   Val Gly Val Ala Pro Gly
   1 5
<210> 8
   <211> 7
   <212> PRT
   <213> artificial sequence
<220>
   <223> synthetic modified peptide
<220>
   <221> MOD_RES
   <222> (1)..(1)
   <223> AMIDATION, Lau (C12)
   sequence description : Lauroyl Xaa Val Gly Val Ala Pro Gly R
<220>
   <221> MISC_FEATURE
   <222> (1)..(1)
   <223> Xaa can be any natural or non-natural aminoacid
<220>
   <221> MISC_FEATURE
   <222> (7)..(7)
   <223> R is OR2 or NR3R4 with R2 =H or methyl, ethyl and with R3 = R4 = H or methyl
<400> 8
   Xaa Val Gly Val Ala Pro Gly
   1 5
<210> 9
   <211> 6
   <212> PRT
   <213> artificial sequence
<220>
   <223> synthetic modified peptide
<220>
   <221> MOD_RES
   <222> (1)..(1)
   <223> AMIDATION, Myr (C14)
   sequence description : Myristoyl Val Gly Val Ala Pro Gly R
<220>
   <221> MISC_FEATURE
   <222> (6)..(6)
   <223> R is OR2 or NR3R4 with R2 =H or methyl, ethyl and with R3 = R4 = H or methyl
<400> 9
   Val Gly Val Ala Pro Gly
   1 5
<210> 10
   <211> 7
   <212> PRT
   <213> artificial sequence
<220>
   <223> synthetic modified peptide
<220>
   <221> MOD_RES
   <222> (1)..(1)
   <223> AMIDATION, Myr (C14)
   sequence description : Myristoyl Xaa Val Gly Val Ala Pro Gly R
<220>
   <221> misc_feature
   <222> (1)..(1)
   <223> Xaa can be any naturally occurring amino acid
<220>
   <221> MISC_FEATURE
   <222> (7)..(7)
   <223> R is OR2 or NR3R4 with R2 =H or methyl, ethyl and with R3 = R4 = H or methyl
<400> 10
   Xaa Val Gly Val Ala Pro Gly
   1 5
<210> 11
   <211 > 6
   <212> PRT
   <213> artificial sequence
<220>
   <223> synthetic modified peptide
<220>
   <221> MOD_RES
   <222> (1)..(1)
   <223> AMIDATION, Ste (C18)
   sequence description : Stearoyl Val Gly Val Ala Pro Gly R
<220>
   <221> MISC_FEATURE
   <222> (6)..(6)
   <223> R is OR2 or NR3R4 with R2 =H or methyl, ethyl and with R3 = R4 = H or methyl
<400> 11
   Val Gly Val Ala Pro Gly
   1 5
<210> 12
   <211> 7
   <212> PRT
   <213> artificial sequence
<220>
   <223> synthetic modified peptide
<220>
   <221> MOD_RES
   <222> (1)..(1)
   <223> AMIDATION, Ste (C18)
   sequence description : Stearoyl Xaa Val Gly Val Ala Pro Gly R
<220>
   <221> MISC_FEATURE
   <222> (1)..(1)
   <223> Xaa can be any natural or non-natural aminoacid
<220>
   <221> MISC_FEATURE
   <222> (7)..(7)
   <223> R is OR2 or NR3R4 with R2 =H or methyl, ethyl and with R3 = R4 = H or methyl
<400> 12
   Xaa Val Gly Val Ala Pro Gly
   1 5
<210> 13
   <211> 6
   <212> PRT
   <213> artificial sequence
<220>
   <223> synthetic modified peptide
<220>
   <221> MOD_RES
   <222> (1)..(1)
   <223> AMIDATION, OI (C18:1)
   sequence description : Oleoyl Val Gly Val Ala Pro Gly R
<220>
   <221> MISC_FEATURE
   <222> (6)..(6)
   <223> R is OR2 or NR3R4 with R2 =H or methyl, ethyl and with R3 = R4 = H or methyl
<400> 13
   Val Gly Val Ala Pro Gly
   1 5
<210> 14
   <211> 7
   <212> PRT
   <213> artificial sequence
<220>
   <223> synthetic modified peptide
<220>
   <221> MOD_RES
   <222> (1)..(1)
   <223> AMIDATION, OI (C18:1)
   sequence description : Oleoyl Xaa Val Gly Val Ala Pro Gly R
<220>
   <221> MISC_FEATURE
   <222> (1)..(1)
   <223> Xaa can be any natural or non-natural aminoacid
<220>
   <221> MISC_FEATURE
   <222> (7)..(7)
   <223> R is OR2 or NR3R4 with R2 =H or methyl, ethyl and with R3 = R4 = H or methyl
<400> 14
   Xaa Val Gly Val Ala Pro Gly
   1 5
<210> 15
   <211> 6
   <212> PRT
   <213> artificial sequence
<220>
   <223> synthetic modified peptide
<220>
   <221> MOD_RES
   <222> (1)..(1)
   <223> AMIDATION, Ar (C20)
   sequence description : Arachidoyl Val Gly Val Ala Pro Gly R
<220>
   <221> MISC_FEATURE
   <222> (6)..(6)
   <223> R is OR2 or NR3R4 with R2 =H or methyl, ethyl and with R3 = R4 = H or methyl
<400> 15
   Val Gly Val Ala Pro Gly
   1 5
<210> 16
   <211> 7
   <212> PRT
   <213> artificial sequence
   <220>
   <223> synthetic modified peptide
<220>
   <221> MOD_RES
   <222> (1)..(1)
   <223> AMIDATION, Ar (C20)
   sequence description : Arachidoyl Xaa Val Gly Val Ala Pro Gly R
<220>
   <221> MISC_FEATURE
   <222> (1)..(1)
   <223> Xaa can be any natural or non-natural aminoacid
<220>
   <221> MISC_FEATURE
   <222> (7)..(7)
   <223> R is OR2 or NR3R4 with R2 =H or methyl, ethyl and with R3 = R4 = H or methyl
<400> 16
   Xaa Val Gly Val Ala Pro Gly
   1 5
<210> 17
   <211> 6
   <212> PRT
   <213> artificial sequence
<220>
   <223> synthetic modified peptide
<220>
   <221> MOD_RES
   <222> (1)..(1)
   <223> AMIDATION, Lin (C18:2)
   sequence description : Linoleoyl Val Gly Val Ala Pro Gly R
<220>
   <221> MISC_FEATURE
   <222> (6)..(6)
   <223> R is OR2 or NR3R4 with R2 =H or methyl, ethyl and with R3 = R4 = H or methyl
<400> 17
   Val Gly Val Ala Pro Gly
   1 5
<210> 18
   <211> 7
   <212> PRT
   <213> artificial sequence
<220>
   <223> synthetic modified peptide
<220>
   <221> MISC_FEATURE
   <222> (1)..(1)
   <223> Xaa can be any natural or non-natural aminoacid
<220>
   <221> MOD_RES
   <222> (1)..(1)
   <223> AMIDATION, Lin (C18:2)
   sequence description : Linoleoyl Xaa Val Gly Val Ala Pro Gly R
<220>
   <221> MISC_FEATURE
   <222> (7)..(7)
   <223> R is OR2 or NR3R4 with R2 =H or methyl, ethyl and with R3 = R4 = H or methyl
<400> 18
   Xaa Val Gly Val Ala Pro Gly
   1 5
<210> 19
   <211> 6
   <212> PRT
   <213> artificial sequence
<220>
   <223> synthetic modified peptide
<220>
   <221> MOD_RES
   <222> (1)..(1)
   <223> AMIDATION, Ela (C18:1)
   sequence description : Elaïdoyl Val Gly Val Ala Pro Gly R
<220>
   <221> MISC_FEATURE
   <222> (6)..(6)
   <223> R is OR2 or NR3R4 with R2 =H or methyl, ethyl and with R3 = R4 = H or methyl
<400> 19
   Val Gly Val Ala Pro Gly
   1 5
<210> 20
   <211> 7
   <212> PRT
   <213> artificial sequence
<220>
   <223> synthetic modified peptide
<220>
   <221> MOD_RES
   <222> (1)..(1)
   <223> AMIDATION, Pal (C16)
   sequence description : Palmitoyl Ala Val Gly Val Ala Pro Gly R
<220>
   <221> MISC_FEATURE
   <222> (7)..(7)
   <223> R is OR2 or NR3R4 with R2 =H or methyl, ethyl and with R3 = R4 = H or methyl
<400> 20
   Ala Val Gly Val Ala Pro Gly
   1 5
<210> 21
   <211> 8
   <212> PRT
   <213> artificial sequence
<220>
   <223> synthetic modified peptide
<220>
   <221> MOD_RES
   <222> (1)..(1)
   <223> ACETYLATION
   sequence description : Acetyl Ile Ala Val Gly Val Ala Pro Gly R
<220>
   <221> MISC_FEATURE
   <222> (8)..(8)
   <223> R is OR2 or NR3R4 with R2 =H or methyl, ethyl and with R3 = R4 = H or methyl
<400> 21
   Ile Ala Val Gly Val Ala Pro Gly
   1 5
<210> 22
   <211> 7
   <212> PRT
   <213> artificial sequence
<220>
   <223> synthetic modified peptide
<220>
   <221> MOD_RES
   <222> (1)..(1)
   <223> ACETYLATION
   sequence description : Acetyl Ser Val Gly Val Ala Pro Gly R
<220>
   <221> MISC_FEATURE
   <222> (7)..(7)
   <223> R is OR2 or NR3R4 with R2 =H or methyl, ethyl and with R3 = R4 = H or methyl
<400> 22
   Ser Val Gly Val Ala Pro Gly
   1 5